Europäisches Patentamt

European Patent Office

Office européen des brevets

(11) Numéro de publication: **0 546 883 B1**

# FASCICULE DE BREVET EUROPEEN

(45) Date de publication du fascicule du brevet: **16.08.95** (51) Int. Cl.⁶: **B01J 23/86**, C07C 17/20

(21) Numéro de dépôt: **92403204.8**

(22) Date de dépôt: **27.11.92**

(54) **Catalyseurs massiques à base d'oxydes de chrome et de nickel, et leur application à la fluoration d'hydrocarbures halogénés.**

(30) Priorité: **09.12.91 FR 9115228**

(43) Date de publication de la demande:
**16.06.93 Bulletin 93/24**

(45) Mention de la délivrance du brevet:
**16.08.95 Bulletin 95/33**

(84) Etats contractants désignés:
**BE DE ES FR GB GR IT NL**

(56) Documents cités:
**EP-A- 0 055 652**
**EP-A- 0 128 510**
**FR-A- 2 276 098**
**FR-A- 2 407 021**
**US-A- 2 435 551**

(73) Titulaire: **ELF ATOCHEM S.A.**
**4 & 8, Cours Michelet**
**La Défense 10**
**F-92800 Puteaux (FR)**

(72) Inventeur: **Cheminal, Bernard**
**"La Rivière",**
**Orliénas**
**F-69530 Brignais (FR)**
Inventeur: **Garcia, François**
**La Chataigneraie,**
**15, Avenue de Gadagne**
**F-69230 Saint Genis Laval (FR)**
Inventeur: **Lacroix, Eric**
**52, Rue Laennec**
**F-69008 Lyon (FR)**
Inventeur: **Lantz, André**
**Domaine de la Hêtraie**
**F-69390 Vernaison (FR)**

(74) Mandataire: **Leboulenger, Jean et al**
**ATOCHEM**
**Département Propriété Industrielle**
**F-92091 Paris la Défense 10 Cédex 42 (FR)**

EP 0 546 883 B1

**Description**

La présente invention concerne la fluoration d'hydrocarbures halogénés par catalyse en phase gazeuse et a plus particulièrement pour objet de nouveaux catalyseurs massiques à base de chrome et de nickel et leur application à la synthèse d'hydrohalogénoalcanes.

Les recherches intenses actuellement menées sur les substituts aux chlorofluorocarbures (CFC) s'orientent, entre autres, vers la synthèse d'hydrohalogénoalcanes. Certaines étapes de cette synthèse peuvent être réalisées par fluoration, en catalyse phase gazeuse hétérogène, avec l'acide fluorhydrique.

De très nombreux composés métalliques (par exemple chrome, cobalt, nickel, fer, cuivre, manganèse,...) présentent un effet catalytique pour ces réactions de fluoration. Les catalyseurs proposés dans la littérature sont soit massiques, soit supportés, le support étant principalement du charbon ou de l'alumine (transformée partiellement en $AlF_3$ après fluoration).

Dans cette seconde catégorie, on trouve une grande variété de composés métalliques et nombreux sont les brevets décrivant des procédés de fluoration basés sur ce type de catalyseurs. Ainsi, on peut citer les brevets US 2 744 147 et US 2 744 148 qui décrivent la fluoration d'un haloalcane sur un catalyseur à base d'un métal (chrome, cobalt, nickel pour le brevet US 2 744 147 et chrome, cobalt, nickel, cuivre, palladium pour le brevet US 2 744 148) supporté sur alumine.

Plus récemment, le brevet EP 0 366 797 décrit un procédé de fluoration utilisant un catalyseur constitué d'au moins un fluorure métallique (nickel, cobalt, fer, manganèse, chrome, cuivre et argent) supporté sur une alumine présentant une mésoporosité importante.

Pour tous ces catalyseurs, le support leur confère une certaine solidité. Cependant, la quantité de matière active étant plus faible que dans un catalyseur massique, l'activité catalytique peut en être affectée. Par ailleurs, ces faibles teneurs en métaux non précieux ne permettent pas d'envisager, de manière rentable, leur récupération dans les catalyseurs usagés.

Les catalyseurs massiques de fluoration sont principalement à base de chrome et les matières premières servant à leur élaboration sont très variées (sels, oxydes, halogénures,...).

Ainsi les brevets US 4 912 270 et EP 0 313 061 revendiquent des procédés de fluoration avec des catalyseurs à base d'oxyde de chrome obtenus respectivement par réduction du trioxyde de chrome au moyen d'un alcool et par pyrolyse du bichromate d'ammonium.

Le brevet FR 2 135 473 décrit la préparation d'un catalyseur au chrome et au nickel, ainsi que son emploi dans la fluoration de composés perhalogénés fonctionnels. Ce catalyseur est obtenu par décomposition thermique de sels organiques de chrome et de nickel. Les teneurs en nickel restent faibles puisque le ratio atomique Ni/Ni + Cr est toujours inférieur à 0,1.

Les publications de brevet JP 2-172932/90 et 2-172933/90 décrivent respectivement la fluoration du difluoro-1,1 trichloro-1,2,2 éthane (F122) et du chloro-1 trifluoro-2,2,2 éthane (F133a) sur un catalyseur au chrome additionné d'un métal dopant qui permet d'abaisser la température de réaction tout en maintenant une activité importante et donc améliore la durée de vie du catalyseur par limitation de la cristallisation du chrome. L'association chrome-nickel n'est pas illustrée par les exemples de ces publications.

Le brevet FR 2 501 062 décrit la préparation d'un oxyde de chrome massique sous forme de microbilles de diamètre compris entre 0,1 et 3 mm. Ce catalyseur est obtenu par gélification d'un sol d'hydroxyde de chrome dans un solvant non miscible ou partiellement miscible à l'eau. Le produit obtenu est très solide et est particulièrement adapté aux réactions de fluoration en lit fluidisé.

L'inconvénient des catalyseurs à base d'oxyde de chrome est leur faible résistance à haute température (350-500°C) à la cristallisation qui contribue à diminuer leur durée de vie.

Par ailleurs, ces catalyseurs au chrome favorisent l'oxydation de l'acide chlorhydrique par l'oxygène dissous dans les réactifs ou introduit volontairement. Par réaction de Deacon (Chemical Week 1987, 24 Juin, page 18) il se forme de l'eau et du chlore qui réagissent à leur tour avec les composés organiques, ce qui conduit à la formation de sous-produits non valorisables et, par conséquent, à une baisse des sélectivités.

Il a maintenant été trouvé que l'ajout d'un composé du nickel à un dérivé du chrome pour former un sol d'hydroxydes de chrome et de nickel permet, tout en gardant les avantages d'un catalyseur massique, non seulement de prolonger la durée de vie du catalyseur par amélioration de la résistance à la cristallisation du composé à base de chrome, mais aussi d'améliorer les sélectivités dans les réactions de fluoration en phase gazeuse grâce à une inhibition partielle de l'oxydation de l'acide chlorhydrique en présence de chrome.

L'invention a donc pour objet des catalyseurs massiques à base d'oxydes de chrome et de nickel, obtenus par un procédé consistant essentiellement :

**a)** à former un sol d'hydroxydes de chrome III et de nickel II.

2

**b)** à gélifier ce sol, et

**c)** à sécher et calciner le produit jusqu'à une température comprise entre 250 et 450°C.

L'invention a également pour objet l'application de ces catalyseurs à la fluoration par HF en phase gazeuse d'hydrocarbures halogénés, saturés ou oléfiniques.

Dans les catalyseurs selon l'invention qui peuvent se présenter sous différentes formes (billes, extrudés, pastilles,...) le rapport atomique Ni/Cr peut aller de 0,05 à 5. Il est avantageusement compris entre 0,1 et 3,5, de préférence entre 0,15 et 3.

En fonction de la technique de gélification mise en oeuvre (sous forme de gouttelettes ou en masse), on obtient le précurseur du catalyseur, constitué d'un mélange homogène d'hydroxydes de chrome et de nickel qui, après séchage, se présente soit sous la forme de microbilles, soit comme une poudre qui peut être mise en forme suivant des techniques bien connues, par exemple par extrusion ou par pastillage. Après calcination, on obtient un catalyseur massique constitué d'un mélange homogène d'oxydes de chrome et de nickel.

Le sol d'hydroxydes de chrome III et de nickel II, peut être formé de façon connue en soi à partir de précurseurs de chrome et de nickel.

Comme précurseurs du chrome, on peut mentionner les oxydes, hydroxydes, halogénures, oxyhalogénures, nitrates, acétates et sulfates de chrome, mais on peut aussi utiliser tout autre composé du chrome susceptible de conduire à un sol d'hydroxyde de chrome. Les précurseurs préférés sont les sels de chrome comme les sulfates, chlorures, acétates et nitrates, le sulfate ou l'acétate de chrome III étant plus particulièrement préférés.

Comme précurseurs du nickel, on peut mentionner les hydroxydes, oxyhalogénures, nitrate, acétate et sulfates de ce métal, mais on peut aussi utiliser tout autre composé du nickel soluble dans l'eau et susceptible de former un sol ou de s'insérer dans le gel formé par le chrome. Les précurseurs préférés sont les sels très solubles comme les nitrates et surtout les chlorures ou sulfates de nickel.

Avec certains précurseurs de chrome et de nickel, notamment les oxydes, hydroxydes, acétates et sulfates, le sol peut être formé dès la température ambiante. Par contre, lorsque le précurseur est un nitrate ou un halogénure de chrome la formation du sol nécessite une étape de chauffage à une température comprise entre 60 et 100°C, de préférence entre 80 et 95°C. D'autre part, même lorsque la nature des précurseurs permet de former le sol à basse température, il est avantageux de chauffer la solution des précurseurs à une température comprise entre 60 et 100°C, de préférence entre 80 et 95°C.

On peut encore partaire la formation du sol en ajoutant à la solution aqueuse des précurseurs un agent complexant du chrome et/ou du nickel tel que, par exemple, l'acétate, le sulfate ou le phosphate d'ammonium, en une quantité molaire pouvant aller jusqu'à 5 fois le nombre total de moles des précurseurs de chrome et de nickel.

Divers additifs peuvent être ajoutés au sol pour parfaire les propriétés physico-chimiques et catalytiques du catalyseur final. On peut ainsi ajouter (% par rapport au poids du sol) :

**a)** 2 à 10 % de poudre de $Cr_2O_3$ ou de $Cr_2O_3$, $2H_2O$ séchée au préalable à 300°C pour augmenter la tenue mécanique des microbilles ;

**b)** 0,1 à 3 % d'alumine monohydrate pour accroître la résistance à l'attrition du catalyseur ;

**c)** 5 à 25 % d'hexaméthylène tétramine et/ou d'urée dont la présence conduit, par décomposition à la température de gélification du sol, à un dégagement supplémentaire d'ammoniaque ;

**d)** 1 à 10 % de silice colloïdale pour accroître la porosité du catalyseur (la silice est éliminée lors du traitement par l'acide fluorhydrique sous forme de tétrafluorure de silicium) ;

**e)** d'autres additifs tels que des agents mouillants ( par exemple, lauryldiéthanolamide, monostéarate de polyéthylène glycol) ou des épaississants (par exemple, l'hydroxyméthylcellulose, la cellulose microcristalline) pour améliorer la sphéricité des microbilles.

A partir du sol d'hydroxydes de chrome et de nickel, les catalyseurs mixtes d'oxydes de chrome et de nickel selon l'invention peuvent être obtenus sous la forme de microbilles en gélifiant le sol de la manière suivante :

**1)** Dispersion du sol sous forme de gouttelettes dans un solvant organique non miscible ou peu miscible à l'eau, et gélification à température élevée ;

**2)** Réception des microbilles formées dans le solvant de gélification ou dans une solution d'ammoniaque pour partaire la gélification ;

**3)** Lavage des microbilles à l'ammoniaque diluée et à l'eau pour éliminer les impuretés, et éventuellement séchage.

3

### Première étape

La formation et la gélification des microbilles sont effectuées dans une colonne d'une hauteur comprise entre 1 et 6 m, prolongée par une zone de désengagement et parcourue de bas en haut par un courant de solvant chaud.

Le sol d'hydroxydes de chrome et de nickel est injecté au sommet de la colonne au moyen d'un tube de faible diamètre disposé concentriquement à l'intérieur d'un autre tube de plus grand diamètre, par où arrive à cocourant le solvant refroidi à une température n'excédant pas 30°C (de préférence 25°C) pour éviter une gélification dans les injecteurs.

Le diamètre de l'injecteur et le débit du sol conditionnent la dispersion des gouttelettes et, par suite, la taille finale des microbilles.

Pendant son injection, il convient de maintenir le sol à une température comprise entre 2 et 8°C, de préférence entre 3 et 5°C, de façon à éviter des variations de sa viscosité. Ces variations traduisent une évolution du sol et, par conséquent, celle des propriétés physicochimiques des microbilles.

Il est avantageux de neutraliser partiellement le sol, avant son injection, avec une quantité d'ammoniaque suffisante pour obtenir une viscosité adaptée à la dispersion des billes. Cette quantité dépend de la nature des précurseurs de chrome et de nickel mis en oeuvre ; le rapport molaire $NH_4 OH/Cr + Ni$ est avantageusement inférieur à 2 et de préférence inférieur à 1,5.

Le courant ascendant de solvant chaud (température comprise entre 25 et 140°C) est introduit par le bas de la colonne avec une vitesse ascensionnelle comprise entre 0,1 et 5 m/s. Cette vitesse détermine le temps de séjour des microbilles et, par conséquent, leur degré de gélification.

Dans sa partie supérieure la colonne peut être munie d'une seconde entrée de solvant refroidi à une température n'excédant pas 30°C. Ceci facilite le maintien d'un gradient de température dans la colonne et permet de contrôler la vitesse de gélification.

Le solvant organique, non miscible ou peu miscible à l'eau, peut être choisi parmi les alcools tels que butanol, hexanol, éthyl-2pentanol, éthyl-2 hexanol, ce dernier alcool étant particulièrement préféré.

### Deuxième étape

A leur sortie à la base de la colonne, les microbilles sont recueillies dans un récepteur contenant le solvant organique de gélification ou, de préférence, contenant de l'ammoniaque dont la concentration peut être comprise entre 0,1 et 14 N et dont la température est maintenue entre 15 et 70°C, de préférence entre 25 et 50°C.

La présence d'ammoniaque dans le récepteur partait efficacement la gélification des microbilles, augmentant ainsi leur tenue mécanique et en assurant également le premier lavage. Une augmentation de la concentration de la solution d'ammoniaque permet d'accroître la solidité et la densité des microbilles.

### Troisième étape

Les microbilles sont lavées plusieurs fois à l'ammoniaque dilué (0,1 à 1 N), puis à l'eau, avant d'être éventuellement séchées à l'air à une température com prise entre 80 et 200°C. Ce séchage à basse température peut cependant être supprimé sans risque d'altération des propriétés des microbilles.

Les microbilles sont ensuite calcinées sous inerte (par exemple, azote,argon,...) ou sous air à une température comprise entre 250 et 450°C, de préférence entre 300 et 420°C.

Au lieu d'être gélifié sous forme de microbilles selon la méthode décrite ci-dessus, le sol d'hydroxydes de chrome et de nickel peut être gélifié complètement par ajout d'une base, de préférence de l'ammoniaque, jusqu'à prise en masse du sol d'hydroxydes. Le produit ainsi obtenu est ensuite lavé à l'ammoniaque dilué (0,1 à 1 N) et/ou à l'eau, avant d'être séché à une température comprise entre 80 et 200°C. Les lavages et filtrations peuvent être facilités par l'addition de 5 à 100 ppm d'un floculant, de préférence un polyacrylamide. Le catalyseur est ensuite calciné comme décrit ci-dessus, cette calcination pouvant être précédée ou suivie d'une mise en forme suivant les techniques connues (extrusion, pastillage).

Les catalyseurs massiques selon l'invention peuvent être utilisés pour la fluoration par HF en phase gazeuse d'hydrocarbures halogénés, saturés ou oléfiniques. Ils conviennent particulièrement bien à la fluoration d'hydrocarbures halogénés conduisant à des composés fluorés en $C_1$ à $C_3$ contenant un ou plusieurs atomes d'hydrogène. Comme exemples d'hydrocarbures halogénés de départ, on peut mentionner, à titre non limitatif, les composés suivants : $CHCl_3$, $CCl_2 = CHCl$, $CHCl_2\text{-}CClF_2$, $CH_2Cl\text{-}CF_3$, $CH_3\text{-}CCl_2\text{-}CH_3$, $CCl_3\text{-}CF_2\text{-}CH_3$, $CCl_3\text{-}CF_2\text{-}CHCl_2$, $CCl_3\text{-}CF_2\text{-}CH_2Cl$, $CHCl_2\text{-}CHCl\text{-}CH_3$, $CH_2Cl\text{-}CHCl\text{-}CH_3$, ainsi que $CCl_2 = CCl_2$ ; ce dernier composé ne contient pas d'hydrogène, mais l'addition d'HF conduit à des composés hydrohalogénés.

Pour travailler à l'activité optimum, le catalyseur nécessite un traitement à l'acide fluorhydrique, dilué ou non avec de l'azote. Bien que la présence de nickel retarde la cristallisation du catalyseur, une telle activation peut générer localement des températures supérieures à 500°C. C'est pourquoi, il est recommandé de contrôler l'exothermicité de l'activation en jouant sur l'ajout d'un diluant de l'HF et en commençant ce traitement à basse température (150-250°C). Par contre, après passage des "vagues d'exothermicité" dans le lit catalytique, il est conseillé de monter progressivement la température pour atteindre un maximum de 350-450°C en fin d'activation.

La température de réaction de fluoration dépend de la réaction étudiée et bien évidemment des produits de réaction désirés. Ainsi, pour une substitution partielle des atomes de chlore par le fluor, on travaille à des températures comprises entre 50 et 350°C, la substitution de la totalité des atomes de chlore pouvant nécessiter des températures comprises entre 300 et 500°C.

Le temps de contact dépend également de la réaction étudiée et des produits recherchés. Généralement, il est compris entre 3 et 100 secondes ; cependant, afin d'obtenir de bons compromis entre taux de conversion et productivité élevés, ce temps dé contact reste généralement inférieur à 30 secondes.

Le rapport molaire HF/composé organique est également lié à la réaction étudiée : il dépend entre autres de la stoechiométrie de la réaction. Dans la majorité des cas, il peut varier entre 1/1 et 20/1, mais, là aussi, afin d'obtenir des productivités élevées, il est souvent inférieur à 10.

La pression de travail est de préférence comprise entre 1 et 20 bars absolus (0,1 à 2 MPa).

Les catalyseurs selon l'invention peuvent travailler en lit fixe ou en lit fluidisé. Les catalyseurs sous forme de microbilles sont très solides et donc particulièrement bien adaptés aux réactions en lit fluidisé.

Les catalyseurs dont l'activité a chuté par suite d'un encrassement, peuvent être régénérés par balayage du catalyseur avec un composé susceptible d'oxyder et de transformer les produits (organiques, coke, ...) déposés sur le catalyseur en produits volatils. A ce titre, l'oxygène ou un mélange contenant de l'oxygène (air par exemple) convient parfaitement et permet de restaurer l'activité initiale du catalyseur.

Afin d'assurer la régénération du catalyseur sans induire une cristallisation, il est recommandé d'effectuer ce traitement à une température comprise entre 200 et 450°C et plus particulièrement entre 300 et 380°C. De même, il convient de imiter l'exothermicité de cette "combustion" en contrôlant le débit d'oxygène (en début de régénération, faible débit d'oxygène dilué dans un inerte) de façon à maintenir une température inférieure à 450°C.

Pour maintenir l'activité du catalyseur, il est également possible d'effectuer la réaction de fluoration en présence d'oxygène introduit dans un rapport molaire $O_2$/composé organique pouvant aller de 0,001 à 0,05 et, de préférence, compris entre 0,005 et 0,03.

Les exemples suivants illustrent l'invention sans la limiter. Le volume poreux indiqué dans les exemples 1 à 12 a été déterminé par porosimétrie mercure et correspond à celui des pores de rayon compris entre 4 nm et 63 μm.

# PREPARATION DES CATALYSEURS
## EXEMPLE 1 Catalyseur A

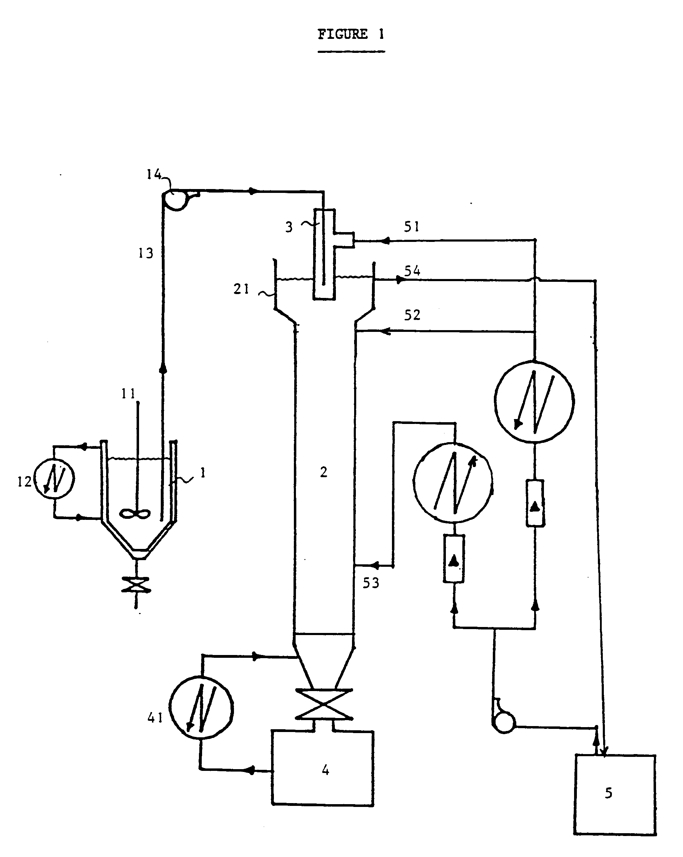

On opère dans l'appareillage représenté sur la figure 1 annexée et comprenant les principaux éléments suivants :

- un réacteur en verre (1) d'un volume de 3 litres pour la préparation du sol d'hydroxydes de chrome et de nickel ; il est muni d'une double enveloppe et équipé d'un agitateur (11) bicônique tournant à 3000 tr/min ; une pompe et un échangeur thermique (12) permettent d'éliminer les calories de la neutralisation et de maintenir ainsi le sol à la température désirée ;
- une colonne en verte (2) pour la gélification du sol sous forme de microbilles ; cette colonne (80 mm de diamètre et 1,5 m de haut) est prolongée à son sommet par une zone de désengagement (21) de 100 mm de diamètre et de 100 mm de haut ;
- un dispositif injecteur (3) consistant d'un tube de 2 mm de diamètre intérieur pour l'injection du sol dans la colonne 2 ; ce tube est disposé concentriquement à l'intérieur d'un tube de 12 mm de diamètre intérieur par lequel on injecte de l'éthyl-2 hexanol ;
- un bac récepteur (4) pour recueillir les microbilles formées dans la colonne 2 ; ce bac, d'un volume de 5 litres, contient de l'ammoniaque homogénéisée et maintenue à la température désirée par un circuit comprenant une pompe et un échangeur thermique (41) ;

- un réservoir (5) pour alimenter le dispositif injecteur 3 et la colonne 2 en éthyl-2 hexanol servant de solvant de déshydratation.

### a) Préparation du sol d'hydroxydes de chrome et de nickel

On prépare à température ambiante une solution aqueuse de chrome et de nickel en dissolvant 550 g de sulfate de chrome basique $Cr_2 (SO_4)_2 (OH)_2 Na_2SO_4$ et 238 g de chlorure de nickel hexahydraté $NiCl_2$, $6H_2O$ dans 367 g d'eau. A cette solution aqueuse, on ajoute ensuite 70 g d'oxyde de chrome III cristallisé.

Le mélange est refroidi à 5°C et, tout en maintenant cette température, on ajoute successivement :
- 151,5 g d'ammoniaque 11N froid (5°C)
- une solution aqueuse froide (5°C) composée de 204 g d'hexaméthylènetétramine, 10,8 g d'urée et 300 ml d'eau
- 225 g d'un sol de silice à 30 % dans l'eau (CECASOL de la Société CECA).

### b) Injection du sol et synthèse des microbilles

Le sol d'hydroxydes de chrome et de nickel, préparé à l'étape a), est ensuite acheminé vers la colonne 2 par la conduite (13) au moyen d'une pompe (14). L'emploi d'une pompe péristaltique permet d'éviter toute variation de débit. Pour des longueurs importantes de la conduite d'acheminement 13, il est nécessaire de la gainer pour éviter les pertes frigorifiques.

Le sol à 5°C est injecté à raison de 0,6 l/h au travers du tube de 2 mm de diamètre intérieur, tandis qu'au moyen du tube de 12 mm de diamètre intérieur on injecte par la conduite 51 de l'éthyl-2 hexanol à une température de 25°C, avec un débit de 10 l/h, pour éviter la gélification prématurée du sol dans le dispositif injecteur 3 ; l'extrémité de ce dernier plonge d'environ 5 mm dans le solvant organique. Un flux d'éthyl-2 hexanol à la même température (25°C) peut être envoyé dans la partie supérieure de la colonne par une conduite 52 dont l'entrée est située en-dessous de la zone de désengagement.

A la base de la colonne par la conduite 53, on introduit avec un débit de 45 l/h un autre courant d'éthyl-2 hexanol, réchauffé à une température de 120°C. Ce courant d'éthyl-2 hexanol parcourt la colonne de bas en haut avec un débit ascensionnel d'environ 9 m/h et sort par un trop plein situé au sommet de la zone de désengagement. Les deux flux d'éthyl-2 hexanol permettent d'obtenir dans la colonne un gradient de température, nécessaire pour maîtriser les cinétiques de gélification et de déshydratation du sol.

L'éthyl-2 hexanol sortant de la colonne est renvoyé au réservoir 5 par la conduite 54. Dans un dispositif non représenté sur la figure 1, il est préalablement purifié par lavage à l'eau et distillation pour éliminer le sulfate d'ammonium dissous, les résidus organiques (urée, hexaméthylène tétramine, formol) et l'eau extraite du sol.

A l'extrémité inférieure de la colonne, on recueille les microbilles dans le bac récepteur 4 contenant de l'ammoniaque 1 N homogénéisée et maintenue à 40°C par le circuit fermé 41.

La production est d'environ 100 g/h par injecteur, mais celle-ci peut être augmentée en utilisant plusieurs injecteurs.

Les microbilles sont lavées abondamment à l'ammoniaque diluée (0,1 N), puis à l'eau et séchées à 120°C. Elles sont ensuite calcinées à 420°C sous atmosphère d'azote pendant 4 heures.

Le catalyseur A ainsi obtenu présente les caractéristiques suivantes :
- rapport atomique Ni/Cr = 0,34
- diamètre des microbilles = 0,5 à 2,5 mm
- densité apparente = 1,25 $g/cm^3$
- surface spécifique (BET) = 185 $m^2/g$
- volume poreux (porosimétrie mercure) = 0,05 $cm^3/g$ (4 nm < $r_{pores}$ < 63 $\mu$m)
- résistance à l'écrasement en lit (BCS) = 0,21 MPa

# EXEMPLE 2 Catalyseur B

On opère comme à l'exemple 1, sauf que la solution d'ammoniaque dans le bac récepteur des microbilles a une concentration de 11 N au lieu de 1 N.

Les caractéristiques du catalyseur B ainsi obtenu sont les suivantes :
- rapport atomique Ni/Cr = 0,27
- diamètre des microbilles = 0,5 à 2,5 mm
- densité apparente = 1,4 $g/cm^3$

- surface spécifique (BET) = 102 $m^2$/g
- volume poreux = 0,07 $cm^3$/g
- résistance à l'écrasement en lit (BCS) = 0,74 MPa

## EXEMPLE 3 Catalyseur C

On opère comme à l'exemple 1, mais sans addition de sol de silice. On obtient un catalyseur C ayant les caractéristiques suivantes :
- rapport atomique Ni/Cr = 0,25
- diamètre des microbilles = 0,5 à 2,5 mm
- densité apparente = 1,5 $g/cm^3$
- surface spécifique (BET) = 25 $m^2$/g
- volume poreux = 0,09 $cm^3$/g

## EXEMPLE 4 Catalyseur D

On opère comme à l'exemple 1 en diminuant la teneur en nickel, c'est-à-dire en partant d'une solution aqueuse préparée en dissolvant 550 g de sulfate de chrome basique et 45 g de chlorure de nickel hexahydraté dans 440 g d'eau.

Le catalyseur D ainsi obtenu présente les caractéristiques suivantes :
- rapport atomique Ni/Cr = 0,07
- diamètre des microbilles = 0,5 à 2,5 mm
- densité apparente = 1,38 $g/cm^3$
- surface spécifique (BET) = 187 $m^2$/g
- volume poreux = 0,044 $cm^3$/g

## EXEMPLE 5 Catalyseur E

On opère comme à l'exemple 1 sauf que les microbilles sont calcinées à 350°C au lieu de 420°C.

Le catalyseur E ainsi obtenu présente les caractéristiques suivantes :
- rapport atomique Ni/Cr = 0,37
- diamètre des microbilles = 0,5 à 2,5 mm
- densité apparente = 1,1 $g/cm^3$
- surface spécifique (BET) = 178 $m^2$/g
- volume poreux = 0,09 $cm^3$/g

## EXEMPLE 6 Catalyseur F (comparatif)

On opère comme à l'exemple 1 en supprimant le nickel. La solution aqueuse de départ est constituée uniquement de sulfate basique de chrome (550 g) et d'eau (475 g).

Les caractéristiques de ce catalyseur F, préparé à titre comparatif, sont les suivantes :
- diamètre des microbilles = 0,5 à 2,5 mm
- densité apparente = 1,04 $g/cm^3$
- surface spécifique (BET) = 209 $m^2$/g
- volume poreux = 0,052 $cm^3$/g
- résistance à l'écrasement en lit (BCS) = 0,37 MPa

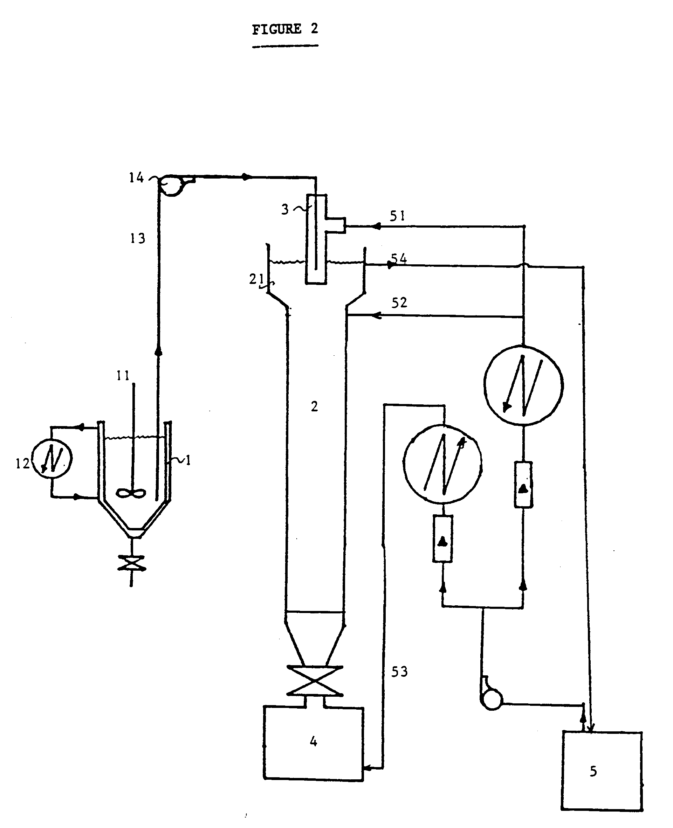

Si l'on remplace l'ammoniaque contenue dans le bac récepteur par de l'éthyl-2 hexanol à 120°C en modifiant l'appareillage conformément à la figure 2 annexée, on obtient un produit présentant les caractéristiques suivantes :
- densité apparente : 1,07 $g/cm^3$
- surface spécifique : 203 $m^2$/g

- résistance à l'écrasement en lit (BCS) = 0,23 MPa

## EXEMPLE 7 Catalyseur G (comparatif)

On opère comme à l'exemple 6 sauf que les microbilles sont calcinées à 350°C au lieu de 420°C.
Le catalyseur ainsi obtenu présente les caractéristiques suivantes :
- diamètre des microbilles = 0,5 à 2,5 mm
- densité apparente = 1,3 g/cm$^3$
- surface spécifique (BET) = 181 m$^2$/g
- volume poreux = 0,05 cm$^3$/g

## EXEMPLE 8 Catalyseur H (comparatif)

On opère comme à l'exemple 1 sans addition de nickel ni de sol de silice. La solution aqueuse de départ est constituée uniquement de sulfate basique de chrome (550 g) et d'eau (475 g).
Les caractéristiques de ce catalyseur H, préparé à titre comparatif, sont les suivantes :
- diamètre des microbilles = 0,5 à 2,5 mm
- densité apparente = 1,4 g/cm$^3$
- surface spécifique (BET) = 10 m$^2$/g
- volume poreux = 0,28 cm$^3$/g

## EXEMPLE 9 Catalyseur I

On prépare une solution de 200 g de nitrate de chrome nonahydraté et de 118,8 g de chlorure de nickel hexahydraté dans 1000 g d'eau. Cette solution est chauffée à 80°C pendant 2 heures, puis gélifiée après refroidissement à 20°C en ajoutant 190 ml d'ammoniaque 14,7N.
Le gel est ensuite lavé à deux reprises avec 450 ml d'ammoniaque 0,1N, puis à deux reprises avec 450 ml d'eau distillée, chaque étape de lavage étant suivie d'une filtration.
La poudre obtenue est alors séchée pendant 14 heures à 100°C sous vide (20 kPa), puis calcinée à 350°C sous atmosphère d'azote pendant 4 heures.
Les caractéristiques du catalyseur I ainsi obtenu sont les suivantes :
- surface spécifique (BET) = 160 m$^2$/g
- volume poreux = 0,74 cm$^3$/g

## EXEMPLE 10 : Catalyseur J (comparatif)

On opère comme à l'exemple 9, mais en chauffant la solution des sels de chrome et de nickel à 40°C au lieu de 80°C.
Le catalyseur J ainsi obtenu présente les caractéristiques suivantes :
- surface spécifique (BET) = 139 m$^2$/g
- volume poreux = 0,60 cm$^3$/g

## EXEMPLE 11 : Catalyseur K

On prépare une solution aqueuse de chrome et de nickel en dissolvant 274 g de chlorure de nickel hexahydraté dans 200 g d'eau, puis en ajoutant 275 g de sulfate de chrome basique et 183,5 g d'eau.
A cette solution, on additionne 112 g de CECASOL, puis 390 ml d'une solution d'ammoniaque 11N jusqu'à prise en masse complète du sol. Le gel obtenu est lavé avec une solution d'ammoniaque diluée et à l'eau avant d'être séché à 120°C pendant 15 heures et calciné à 250°C pendant 4 heures.
La poudre recueillie est additionnée d'une solution aqueuse à 12 % d'alcool polyvinylique, puis séchée à 85°C pendant 15 heures. Le mélange est ensuite mis en forme par pastillage et calciné à 420°C pendant 4 heures.

Le catalyseur K ainsi obtenu présente les caractéristiques suivantes :
Cr = 24,7 % massique
Ni = 20,15 % massique
rapport atomique Ni/Cr = 0,42
surface spécifique (BET) = 54,8 m$^2$/g
volume poreux = 0,162 cm$^3$/g

## EXEMPLE 12 : Catalyseur L (comparatif)

On prépare une solution de chrome et nickel en mélangeant à température ambiante 80 ml d'une solution 1 M de Cr(NO$_3$)$_3$, 9H$_2$O et 80 ml d'une solution 1 M de NiCl$_2$, 6H$_2$O. Le mélange est ensuite neutralisé par 50 ml d'ammoniaque 14 N et le produit, récupéré par centrifugation, est séché à 100°C pendant 15 heures.

La poudre obtenue est additionnée de 0,4 g de graphite et 5,1 g d'une solution aqueuse à 12 % d'alcool polyvinylique, puis séchée à 100°C (15 heures) et mise sous forme de pastilles. Le produit final est calciné à 350°C pendant 4 heures.

Le catalyseur L présente les caractéristiques suivantes :
rapport atomique Ni/Cr = 0,95
surface spécifique (BET) = 82,8 m$^2$/g
volume poreux = 0,17 cm$^3$/g

## EXEMPLES DE FLUORATION

Dans les exemples suivants :
- les pourcentages indiqués sont des pourcentages molaires ;
- l'acide fluorhydrique utilisé est un produit commercial ne contenant que des traces d'eau ;
- le chloro-1 trifluoro-2,2,2 éthane (F133a) de départ est un produit pur à 99,9 % ;
- le réacteur utilisé est un tube en Inconel de 250 ml chauffé par l'intermédiaire d'un bain fluidisé d'alumine.

L'activation du catalyseur par HF est réalisée dans ce réacteur sur un échantillon de 100 ml. Après un séchage de 3 heures sous azote (5 l/h) à 250°C, l'acide fluorhydrique est additionné progressivement, à cette même température, à l'azote, pendant une durée de 5 heures (2 moles d'HF introduites en cinq heures). Après passage des pics d'exothermicité, le débit d'HF est augmenté pour atteindre 1 mole/h, puis la température est portée à 350°C. Un palier de température est observé dans ces conditions pendant 8 heures.

Avant leur introduction dans le réacteur, les réactifs sont mélangés et chauffés à la température de réaction dans un préchauffeur en Inconel.

Après lavage à l'eau -afin d'éliminer les hydracides- et séchage sur CaCl$_2$, les produits de réaction sont analysés en ligne, par chromatographie en phase gazeus

## EXEMPLES 13 A 15

On effectue des tests de fluoration du F133a sous pression atmosphérique, en absence d'oxygène, avec les catalyseurs A, D et F, activés selon la procédure décrite précédemment. Les résultats de fluoration sont résumés dans le tableau 1.

On constate que l'activité du catalyseur F (chrome seul) chute beaucoup plus rapidement que celle des catalyseurs A et D (nickel-chrome) et que les sélectivités sont aussi moins bonnes.

## TABLEAU 1

| CATALYSEUR | A Exemple 13 | | D Exemple 14 | | F comparatif Exemple 15 | |
|---|---|---|---|---|---|---|
| Age du catalyseur (heures) | 26 | 221 | 22 | 220 | 24 | 221 |
| Rapport molaire HF/F133a | 4,1 | 4,2 | 3,8 | 3,9 | 4,0 | 4,0 |
| Temps de contact (s) | 3,9 | 3,8 | 4,1 | 4,0 | 3,9 | 3,9 |
| Température (°C) | 350 | 350 | 350 | 350 | 350 | 350 |
| Taux de transformation du F133a (%) | 22,4 | 22,6 | 20,2 | 18,2 | 19,5 | 9,1 |
| Sélectivité 134a ($CF_3CH_2F$) (%) | 97,3 | 97,3 | 97,9 | 98,3 | 96,9 | 94,5 |
| Sélectivité F1122 ($CF_2=CHCl$) (%) | 1,3 | 1,3 | 1,0 | 1,1 | 1,5 | 3,3 |
| Sélectivité F123 ($CF_3CHCl_2$) (%) | 0 | 0 | 0 | 0 | 0,3 | 1,1 |
| Sélectivité F124 ($CF_3CHClF$) (%) | 0,3 | 0,3 | 0,2 | 0,3 | 0,5 | 0,5 |
| Sélectivité F125 ($CF_3CHF_2$) (%) | 0,1 | 0 | 0 | 0 | 0 | 0 |
| Sélectivité F143a ($CF_3CH_3$) (%) | 0,3 | 0,3 | 0,2 | 0,3 | 0,3 | 0 |

## EXEMPLES 16 ET 17

On effectue des tests de fluoration du F133a, sous une pression absolue de 1,5 MPa, en présence d'oxygène par injection d'air et avec les catalyseurs E et G activés selon la procédure décrite précédemment. Les résultats de fluoration sont résumés dans le tableau 2.

## TABLEAU 2

| CATALYSEUR | E Exemple 16 | | G comparatif Exemple 17 | |
|---|---|---|---|---|
| Age du catalyseur (heures) | 22 | 268 | 22 | 265 |
| Rapport molaire HF/F133a | 5,1 | 5,1 | 5,1 | 5,1 |
| Rapport molaire $O_2$/F133a | 0,01 | 0,01 | 0,01 | 0,02 |
| Temps de contact (s) | 19,6 | 19,6 | 19,6 | 19,7 |
| Température (°C) | 350 | 350 | 350 | 350 |
| Taux de transformation du F133a (%) | 23,2 | 24,7 | 21,6 | 19,7 |
| Sélectivité F134a (%) | 96,5 | 95,9 | 93,3 | 89,8 |
| Sélectivité F1122 (%) | 0,05 | 0,04 | 0,1 | 0,1 |
| Sélectivité F123 (%) | 0,5 | 0,4 | 2,1 | 3 |
| Sélectivité F124 (%) | 0,5 | 0,9 | 1,1 | 1,3 |
| Sélectivité F125 (%) | 0,2 | 0,4 | 0,3 | 0,2 |
| Sélectivité F143a (%) | 0 | 0,02 | 0,1 | 0,1 |

Tout comme précédemment le catalyseur E (Ni-Cr) (exemple 16) donne lieu à une activité plus stable et à une meilleure sélectivité que le catalyseur G sans nickel (exemple 17).

## EXEMPLES 18, 19 ET 20

Ces exemples, résumés dans le tableau 3 suivant, ont été réalisés avec les catalyseurs C et H sans silice et permettent de comparer les résultats de fluoration du F133a sous pression atmosphérique, en absence d'oxygène.

Après 548 heures d'utilisation, le catalyseur C de l'exemple 19 a fait l'objet d'une régénération par traitement sous air (1 mole/heure) à 300 °C pendant 24 heures. Le catalyseur C ainsi régénéré a ensuite été utilisé pour les essais de l'exemple 20.

L'examen des résultats permet d'apprécier l'effet du nickel sur l'activité du catalyseur, malgré l'absence de silice.

On constate en outre que le catalyseur C régénéré (exemple 20) présente une activité comparable à celle du catalyseur C en début d'essai.

EP 0 546 883 B1

**TABLEAU 3**

| CATALYSEUR | H comparatif Exemple 18 | C Exemple 19 | | | C régénéré Exemple 20 | |
|---|---|---|---|---|---|---|
| Age du catalyseur (heures) | 47 | 65 | 333 | 548 | 48(*) | 245 (*) |
| Rapport molaire HF/F133a | 3,9 | 4 | 4 | 4 | 4 | 4 |
| Temps de contact (s) | 4 | 4,1 | 4,1 | 4,2 | 4,8 | 4,6 |
| Température (°C) | 350 | 350 | 350 | 350 | 350 | 350 |
| Taux de transformation du F133a (%) | 4 | 20,4 | 18,6 | 14,8 | 20,4 | 20,8 |
| Sélectivité F134a (%) | 87,5 | 97,1 | 97,3 | 98 | 98,5 | 98,1 |
| Sélectivité F1122 (%) | 5 | 1 | 1,1 | 1,4 | 1 | 1 |
| Sélectivité F123 (%) | 2,5 | 0,5 | 0,3 | 0,3 | 0 | 0 |
| Sélectivité F124 | 1,3 | 0,5 | 0,3 | 0,3 | 0,2 | 0,2 |
| Sélectivité F125 (%) | 0 | 0 | 0 | 0 | 0 | 0 |
| Sélectivité F143a (%) | 1,3 | 0 | 0 | 0 | 0 | 0 |

(*) Après régénération

## EXEMPLE 21

Cet exemple, résumé dans le tableau 4 suivant, a été réalisé à pression atmosphérique, en l'absence d'oxygène, avec les catalyseurs I et J préparés à partir de sols chauffés respectivement à 80 et 40°C.

## TABLEAU 4

| CATALYSEUR | I | J (comparatif) |
|---|---|---|
| Age du catalyseur (heures) | 28 | 21 |
| Rapport molaire HF/F133a | 4,4 | 4,3 |
| Temps de contact (s) | 3,8 | 3,9 |
| Température (°C) | 350 | 350 |
| Taux de transformation du F133a (%) | 21,2 | 16,3 |
| Sélectivité F134 a (%) | 98,3 | 97,5 |
| Sélectivité F1122 (%) | 0,9 | 1,5 |
| Sélectivité F123 (%) | 0 | 0,1 |
| Sélectivité F124 (%) | 0,2 | 0,2 |
| Sélectivité F125 (%) | 0,2 | 0,1 |
| Sélectivité F143a (%) | 0,3 | 0,6 |

L'examen des résultats permet d'apprécier l'influence de la température de préparation du sol de chrome et de nickel sur l'activité du catalyseur.

**Revendications**

1. Catalyseurs massiques à base d'oxydes de chrome et de nickel, caractérisés en ce que le rapport atomique Ni/Cr est compris entre 0,05 et 5 et qu'ils sont obtenus par un procédé consistant essentiellement :
   a) à former un sol d'hydroxydes de chrome III et de nickel II,
   b) à gélifier ce sol, et
   c) à sécher et calciner le produit à une température comprise entre 250 et 450°C.

2. Catalyseurs selon la revendication 1, dans lesquels le rapport atomique Ni/Cr est compris entre 0,1 et 3,5, de préférence entre 0,15 et 3.

3. Catalyseur selon la revendication 1 ou 2, caractérisé en ce que le sol d'hydroxydes de chrome et de nickel est obtenu à partir de sulfate, acétate ou nitrate de chrome III et de chlorure, sulfate ou nitrate de nickel.

EP 0 546 883 B1

4. Catalyseur selon l'une des revendications 1 à 3, caractérisé en ce que le sol contient en outre au moins un additif choisi parmi les suivants :
   - poudre de Cr$_2$O$_3$ cristallisé
   - alumine monohydrate
   - hexaméthylènetétramine et/ou urée
   - silice colloïdale
   - agents mouillants
   - épaississants

5. Catalyseur selon l'une des revendications 1 à 4, caractérisé en ce que la formation du sol comprend une étape de chauffage à une température comprise entre 60 et 100°C, de préférence entre 80 et 95°C.

6. Catalyseur selon l'une des revendications 1 à 5, caractérisé en ce que le sol est formé en présence d'un agent complexant du chrome et/ou du nickel.

7. Catalyseur selon l'une des revendications 1 à 6, caractérisé en ce que le sol est gélifié au moyen d'ammoniaque.

8. Catalyseur selon l'une des revendications 1 à 7 sous forme de microbilles, caractérisé en ce que la gélification du sol d'hydroxydes de chrome et de nickel est effectuée de la manière suivante :
   (a) dispersion du sol sous forme de gouttelettes dans un solvant organique non miscible ou peu miscible à l'eau, et gélification à température élevée ;
   (b) réception des microbilles formées dans le même solvant organique ou dans une solution d'ammoniaque ;
   (c) lavage des microbilles à l'ammoniaque diluée, puis à l'eau, et éventuellement séchage.

9. Catalyseur selon la revendication 8, caractérisé en ce que le solvant organique est l'éthyl-2 hexanol.

10. Catalyseur selon l'une des revendications 1 à 7, caractérisé en ce qu'il se présente sous forme de pastilles ou d'extrudés.

11. Catalyseur selon l'une des revendications 1 à 10, caractérisé en ce que la calcination est effectuée à une température comprise entre 300 et 420°C.

12. Procédé de fluoration par HF en phase gazeuse d'hydrocarbures halogénés, saturés ou oléfiniques, caractérisé en ce qu'on utilise un catalyseur selon l'une des revendications 1 à 11.

13. Procédé selon la revendication 12, dans lequel l'hydrocarbure halogéné est le chloro-1 trifluoro-2,2,2 éthane.

**Claims**

1. Bulk catalysts based on chromium and nickel oxides, characterized in that the Ni/Cr atomic ratio is between 0.05 and 5 and in that they are obtained by a process consisting essentially:
   a) in forming a sol of chromium(III) and nickel(II) hydroxides,
   b) in gelling this sol, and
   c) in drying and calcining the product at a temperature of between 250 and 450°C.

2. Catalysts according to Claim 1, in which the Ni/Cr atomic ratio is between 0.1 and 3.5 and preferably between 0.15 and 3.

3. Catalyst according to Claim 1 or 2, characterized in that the sol of chromium and nickel hydroxides is obtained from chromium(III) sulphate, acetate or nitrate and from nickel chloride, sulphate or nitrate.

4. Catalyst according to one of Claims 1 to 3, characterized in that the sol additionally contains at least one additive chosen from the following:
   - crystalline Cr$_2$O$_3$ powder

14

- alumina monohydrate
- hexamethylenetetramine and/or urea
- colloidal silica
- wetting agents
- thickeners

5. Catalyst according to one of Claims 1 to 4, characterized in that the formation of the sol comprises a heating stage at a temperature of between 60 and 100°C and preferably between 80 and 95°C.

6. Catalyst according to one of Claims 1 to 5, characterized in that the sol is formed in the presence of a complexing agent for chromium and/or nickel.

7. Catalyst according to one of Claims 1 to 6, characterized in that the sol is gelled by means of aqueous ammonia.

8. Catalyst according to one of Claims 1 to 7 in the form of microbeads, characterized in that gelling of the sol of chromium and nickel hydroxides is carried out in the following way:
    (a) dispersing the sol in the form of droplets in an organic solvent which is immiscible or virtually immiscible with water and gelling at high temperature;
    (b) receiving the microbeads formed in the same organic solvent or in an aqueous ammonia solution;
    (c) washing the microbeads with dilute aqueous ammonia and then with water and optionally drying.

9. Catalyst according to Claim 8, characterized in that the organic solvent is 2-ethylhexanol.

10. Catalyst according to one of Claims 1 to 7, characterized in that it is provided in the form of pellets or extrudates.

11. Catalyst according to one of Claims 1 to 10, characterized in that the calcination is carried out at a temperature of between 300 and 420°C.

12. Process for fluorination by HF in the gas phase of saturated or olefinic halogenated hydrocarbons, characterized in that a catalyst according to one of Claims 1 to 11 is used.

13. Process according to Claim 12, in which the halogenated hydrocarbon is 1-chloro-2,2,2-trifluoroethane.

**Patentansprüche**

1. Vollkatalysatoren auf Basis von Chrom- und Nickeloxiden, dadurch gekennzeichnet, daß das Atomverhältnis von Nickel zu Chrom 0,05 bis 5 beträgt und die Katalysatoren mit einem Verfahren hergestellt werden, das im wesentlichen aus den folgenden Schritten besteht:
    a) Bildung eines Sols aus Chrom(III)- und Nickel(II)-hydroxiden,
    b) Gelierung des Sols, und
    c) Trocknung und Calcinierung des Produkts bei einer Temperatur zwischen 250 und 450 °C.

2. Katalysatoren nach Anspruch 1, wobei das Atomverhältnis von Nickel zu Chrom zwischen 0,1 und 3,5, vorzugsweise zwischen 0,15 und 3 liegt.

3. Katalysatoren nach Anspruch 1 oder 2, dadurch gekennzeichnet, daß das Chrom- und Nickelhydroxid-Sol aus Chrom(III)-sulfat, -acetat oder -nitrat und aus Nickelchlorid, -sulfat oder -nitrat gewonnen wird.

4. Katalysatoren nach einem der Ansprüche 1 bis 3, dadurch gekennzeichnet, daß das Sol zusätzlich wenigstens ein Additiv enthält, ausgewählt aus:
    - Pulver aus kristallisiertem $Cr_2O_3$,
    - Aluminiumoxid-Monohydrat,
    - Hexamethylentetramin und/oder Harnstoff,
    - kolloidalem Siliciumdioxid,
    - Benetzungsmitteln,
    - Verdickungsmitteln.

**5.** Katalysatoren nach einem der Ansprüche 1 bis 4, dadurch gekennzeichnet, daß die Bildung des Sols einen Aufheizschritt auf eine Temperatur zwischen 60 und 100 °C, vorzugsweise zwischen 80 und 95 °C umfaßt.

**6.** Katalysator nach einem der Ansprüche 1 bis 5, dadurch gekennzeichnet, daß das Sol in Gegenwart eines Chrom- und/oder Nickel-Komplexierungsmittels gebildet wird.

**7.** Katalysator nach einem der Ansprüche 1 bis 6, dadurch gekennzeichnet, daß das Sol mittels Ammoniak geliert wird.

**8.** Katalysator nach einem der Ansprüche 1 bis 7, in Form von Mikrokügelchen, dadurch gekennzeichnet, daß die Gelierung des Sols aus Chrom- und Nickelhydroxiden auf folgende Weise durchgeführt wird:
(a) Dispersion des Sols in Form von kleinen Tröpfchen in einem organischen, mit Wasser nicht oder wenig mischbaren Lösungsmittel und Gelierung bei erhöhter Temperatur;
(b) Aufnahme der gebildeten Mikrokügelchen in dem gleichen organischen Lösungsmittel oder in einer ammoniakalischen Lösung;
(c) Waschen der Mikrokügelchen mit verdünntem Ammoniak, anschließend mit Wasser, gegebenenfalls gefolgt von einer Trocknung.

**9.** Katalysator nach Anspruch 8, dadurch gekennzeichnet, daß das organische Lösungsmittel 2-Ethylhexanol ist.

**10.** Katalysator nach einem der Ansprüche 1 bis 7, dadurch gekennzeichnet, daß er in Form von Pellets oder Extrudaten vorliegt.

**11.** Katalysator nach einem der Ansprüche 1 bis 10, dadurch gekennzeichnet, daß die Calcinierung bei einer Temperatur zwischen 300 und 420 °C durchgeführt wird.

**12.** Verfahren zur Fluorierung von gesättigten oder olefinischen halogenierten Kohlenwasserstoffen mit HF in der Gasphase, dadurch gekennzeichnet, daß ein Katalysator nach einem der Ansprüche 1 bis 11 verwendet wird.

**13.** Verfahren nach Anspruch 12, bei dem der halogenierte Kohlenwasserstoff 1-Chlor-2,2,2-trifluorethan ist.

FIGURE 1

FIGURE 2